# EUROPEAN PATENT APPLICATION

(11) **EP 4 206 177 A1**
(43) Date of publication of application: **05.07.2023**
(21) Application number: 21880552.1
(22) Date of filing: 14.10.2021
(51) Int. Cl.: C07C 51/43, C07C 51/48, C07C 59/08, C07C 51/377, C07C 57/04

(54) **METHOD FOR VAPORIZING LACTIC ACID, APPARATUS FOR VAPORIZING LACTIC ACID, AND METHOD FOR PREPARING ACRYLIC ACID**

(30) Priority: 16.10.2020 KR 20200134655; 16.10.2020 KR 20200134656; 06.11.2020 KR 20200148082; 24.12.2020 KR 20200183552
(71) Applicant: Lg Chem, Ltd., Seoul 07336 (KR)
(72) Inventor: IM, Yong O, Daejeon 34122 (KR); JEONG, Hoiin, Daejeon 34122 (KR); CHOI, Inho, Daejeon 34122 (KR); LEE, Jiyoung, Daejeon 34122 (KR); HONG, Daeho, Daejeon 34122 (KR)
(74) Representative: Goddar, Heinz J.
(86) International application number: PCT/KR2021/014268
(87) International publication number: WO 2022/080905

(57) **Abstract**

The present disclosure relates to an apparatus for vaporizing lactic acid. According to the method and apparatus for vaporizing lactic acid of the present disclosure, the content of lactic acid oligomers can be reduced within a short period of time, and the content of lactic acid single-molecule can be increased.

## Description

### [TECHNICAL FIELD]

### CROSS-REFERENCE TO RELATED APPLICATION(S)

This application claims the benefit of Korean Patent Application No. 10-2020-0134656 filed on October 16, 2020, Korean Patent Application No. 10-2020-0134655 filed on October 16, 2020, Korean Patent Application No. 10-2020-0148082 filed on November 6, 2020 and Korean Patent Application No. 10-2020-0183552 filed on December 24, 2020 in the Korean Intellectual Property Office, the disclosures of which are incorporated herein by reference in their entirety.

The present disclosure relates to a method for vaporizing lactic acid, an apparatus for vaporizing lactic acid, and a method for preparing acrylic acid from lactic acid.

### [BACKGROUND ART]

Lactic acid is also known as milk acid, or lactate, which is an organic acid with a relatively simple structure that contains both a hydroxy group and a carboxyl group in a molecule.

Traditionally, lactic acid is naturally produced during fermentation processes of lactose, glucose or the like, and plays the role in improving the flavor of fermented foods and is non-toxic to the human body. Thus, lactic acid has been used variously in the food field, such as flavoring agents, preservatives, and pH regulators, the cosmetic field such as moisturizing agents and skin whitening agents, or the medical field such as intravenous injections solution, dialysis solutions, and calcium agents.

Recently, lactic acid polymer and polylactide are biodegradable, and thus attracts increasing attentions as an eco-friendly alternative polymer capable of replacing naturally non-decomposable plastics such as polyolefins, polystyrenes, and polyesters produced from petroleum, and further attract considerable attention as a precursor of acrylic acid, which is considered very important industrially.

Lactic acid can be mainly produced by microbial fermentation or chemical synthesis methods. Recently, methods for producing lactic acid using biomass resources, including starch-based biomass such as corn, sugar-based biomass such as sugar cane, or cellulose-based biomass obtained from woody or herbaceous plants, are being studied.

The lactic acid thus obtained is usually concentrated and stored at a high concentration during storage or distribution process after production. However, due to the structural features peculiar to the lactic acid molecule containing both a hydroxy group and a carboxyl group in the molecule, it often forms a dimer structure, or forms an oligomer having a form in which water molecules are removed and subjected to dehydration condensation.

A large amount of lactic acid is lost by this dimerization or oligomerization. The oligomerized lactic acid has a problem that the amount of by-products generated is increased in the chemical reaction using lactic acid, coke is formed in the reaction process, and the efficiency of the reaction is greatly reduced.

Therefore, when using the concentrated and stored lactic acid as lactic acid itself or charging it into other chemical reactions, it is necessary to reduce the content of lactic acid oligomers. It is known that the chemical equilibrium between lactic acid and lactic acid oligomer involves only the concentration and temperature of lactic acid. Since the equilibrium movement speed is very slow, there is growing interest in pretreatment methods for the use of concentrated lactic acid, i.e., methods of reducing the content of lactic acid oligomers in the feed and increasing the content of lactic acid single-molecule.

### [DETAILED DESCRIPTION OF THE INVENTION]

### [Technical Problem]

It is one object of the present disclosure to provide a method for vaporizing lactic acid and an apparatus for vaporizing lactic acid, that can reduce the content of lactic acid oligomers within a short period of time and can obtain a vaporized lactic acid single-molecule.

It is one object of the present disclosure to provide a method for preparing acrylic acid from the vaporized lactic acid.

### [Technical Solution]

Provided herein is a method for vaporizing lactic acid comprising the steps of: heating and pressurizing a lactic acid aqueous solution having a 1-1 concentration; spraying a 1-1 stream of a liquid phase containing the heated and pressurized lactic acid aqueous solution having a 1-1 concentration; vaporizing the lactic acid contained in the 1-1 stream through spraying; and obtaining a 1-3 stream of a gas phase containing lactic acid molecule.

According to one embodiment of the present disclosure, in the heating and pressurizing step, the lactic acid aqueous solution having the 1-1 concentration may be heated and pressurized under conditions of a temperature of about 150°C to about 250°C, preferably about 150°C or more, or about 160°C or more, about 250°C or less, or about 200°C or less; and a pressure of about 1 bar to about 40 bar, preferably more than about 1 bar, or more than about 5 bar, about 40 bar or less, or about 30 bar or less, or about 15 bar or less.

According to one embodiment of the present disclosure, the 1-1 concentration may be about 20 to about 99 wt.%, about 20 wt.% or more, or about 30 wt.% or more, or about 40 wt.% or more, or about 50 wt.% or more, or about 60 wt.% or more, or about 70 wt.% or more, or about 75 wt.% or more, and about 99 wt.% or less, about 95 wt.% or less, or about 90 wt.% or less, or about 85 wt.% or less. That is, the lactic acid aqueous solution may be concentrated to a high concentration.

According to another embodiment of the present disclosure, the temperature of the 1-1 stream, that is, the temperature of the sprayed 1-1 stream may be about 10 to about 300°C, preferably about 10°C or more, or about 50°C or more, or about 150°C or more, and about 300°C or less, or about 250°C or less, or about 200°C or less.

According to another embodiment of the present disclosure, the 1-1 stream may be preferably sprayed at a flow rate of about 0.1 g/min to 1.0 g/min, preferably about 0.1 g/min or more, or about 0.15 g/min or more, and about 1.0 g/min or less, or about 0.5 g/min or less, or about 0.3 g/min or less. At this time, the temperature inside the reactor may be about 300 to about 400°C.

That is, the 1-1 stream is heated and pressurized under conditions of a temperature of 150°C to 250°C and a pressure of 1 to 40 bar, transported, and then can be sprayed in the form of aerosol into a normal pressure reactor under a temperature condition of about 10°C to about 300°C.

According to one embodiment of the present disclosure, when the 1-1 stream is sprayed, the 1-2 stream of a gas phase containing a steam can be mixed and sprayed.

Further, the temperature of the 1-2 stream may be about 200 to about 600°C, preferably about 250°C or more, or about 300°C or more, or about 350 °C or more, or about 400°C or more, and about 600°C or less, or about 550°C or less, or about 530°C or less.

At this time, the temperature difference between the 1-2 stream and the 1-1 stream may be, preferably, about 200°C or more, or about 250°C or more, and about 500°C or less, or about 450°C or less.

Further, the 1-2 stream may be preferably sprayed at a flow rate of 0.1 g/min to 3.0 g/min, preferably a flow rate of about 0.1 g/min or more, or about 0.2 g/min or more, or about 0.3 g/min or more, and about 3.0 g/min or less, or about 2.0 g/min or less, or about 1.0 g/min or less.

At this time, in the mixing and spraying step, the 1-1 stream flow rate: the 1-2 stream flow rate may be preferably about 1: 1.5 to about 1:5.

On the other hand, provided herein is a method for preparing acrylic acid, comprising the steps of: obtaining lactic acid molecules by vaporizing lactic acid according to any one of the above-mentioned methods; subjecting the lactic acid molecules to a dehydration reaction to prepare acrylic acid; and obtaining the acrylic acid.

On the other hand, provided herein is an apparatus for vaporizing lactic acid, comprising: a pretreatment section that heats and pressurizes a lactic acid aqueous solution having a 1-1 concentration; a feed supply section that receives the supply of the lactic acid aqueous solution from the pretreatment section and supplies a 1-1 stream containing the lactic acid aqueous solution; a spray section that sprays the 1-1 stream transferred from the feed supply section into the vaporization reactor; a vaporization reactor that vaporizes the sprayed lactic acid aqueous solution; and an obtaining section that obtains a 1-3 stream containing vaporized lactic acid molecules.

According to one embodiment of the present disclosure, the pretreatment section may further include a temperature adjusting part and a pressure regulating part that heat and pressurize the lactic acid aqueous solution having the 1-1 concentration under conditions of a temperature of about 150°C to about 250°C, preferably about 150°C or more, or about 160°C or more, and about 250°C or less, or about 200°C or less; and a pressure of about 1 bar to about 40 bar, preferably more than about 1 bar, or about 5 bar or more, and about 40 bar or less, or about 30 bar or less, or about 15 bar or less.

According to one embodiment of the present disclosure, the 1-1 concentration may be about 20 to about 99 wt.%, about 20 wt.% or more, or about 30 wt.% or more, or about 40 wt.% or more, or about 50 wt.% or more, or about 60 wt.% or more, or about 70 wt.% or more, or about 75 wt.% or more, and about 99 wt.% or less, about 95 wt.% or less, or about 90 wt.% or less, or about 85 wt.% or less. That is, the lactic acid aqueous solution may be concentrated to a high concentration.

According to another embodiment of the present disclosure, in the feed supply section, the temperature of the 1-1 stream may be about 150 to about 300°C, preferably about 150°C or more, or about 160°C or more, and about 300°C or less, or about 250°C or less, or about 200°C or less. The feed supply section may further include a feed temperature adjusting part that adjusts the temperature of the 1-1 stream.

According to another embodiment of the present disclosure, the spray section may include a 1-1 stream nozzle that adjusts so that the 1-1 stream is sprayed at a flow rate of about 0.1 g/min to 1.0 g/min, preferably about 0.1 g/min or more, or about 0.15 g/min or more, and about 1.0 g/min or less, or about 0.5 g/min or less, or about 0.3 g/min or less.

According to one embodiment of the present disclosure, the apparatus for vaporizing lactic acid further comprises a steam supply section that supplies steam, and the spray section may further comprise a 1-2 stream nozzle that receives supply of steam from the steam generator and sprays a 1-2 stream of a gas phase containing a steam into the interior of the vaporization reactor.

Further, the temperature of the 1-2 stream may be about 200 to about 600°C, preferably about 250°C or more, or about 300°C or more, or about 350°C or more, or about 400°C or more, and about 600°C or less, or about 550°C or less, or about 530°C or less.

For this purpose, the steam supply section may further include a steam temperature adjusting part that adjusts the temperature of the 1-2 stream.

At this time, the temperature difference between the 1-2 stream and the 1-1 stream may be preferably about 200°C or more, or about 250°C or more, and about 500°C or less, or about 450°C or less.

Further, the spray section may preferably adjust so that the 1-2 stream is sprayed at a flow rate of 0.1 g/min to 3.0 g/min, preferably about 0.1 g/min or more, or about 0.2 g/min or more, or about 0.3 g/min or more, and about 3.0 g/min or less, or about 2.0 g/min or less, or about 1.0 g/min or less.

At this time, the spray section may preferably adjust so that the 1-1 stream flow rate: the 1-2 stream flow rate = 1: 1.5 to 1: 5.

On the other hand, further provided herein is a method for vaporizing lactic acid comprising the steps of: mixing and spraying a 3-1 stream of a liquid phase containing a lactic acid aqueous solution and a 3-2 stream of a gas phase containing a steam; vaporizing the lactic acid aqueous solution through heat exchange between the 3-1 stream and the 3-2 stream; and obtaining a 3-3 stream of a gas phase containing lactic acid single-molecule.

According to one embodiment of the present disclosure, the lactic acid aqueous solution contained in the 3-1 stream may be concentrated to a high concentration so that the lactic acid concentration is about 40 to about 99 wt.%, about 45 wt.% or more, or about 50 wt.% or more, or about 60 wt.% or more, or about 70 wt.% or more, or about 75 wt.% or more, and about 99 wt.% or less, about 95 wt.% or less, or about 90 wt.% or less, or about 85 wt.% or less.

Further, the concentration of a multimer in the lactic acid aqueous solution contained in the 3-1 stream, that is, the concentration of the lactic acid dimer or the lactic acid oligomer of trimer or higher may be about 2 to about 55 wt.%, or about 2 wt.% or more, or about 5 wt.% or more, or about 7 wt.% or more, or about 8 wt.% or more, and about 55 wt.% or less, or about 40 wt.% or less, or about 20 wt.% or less. The content of the multimer may be relatively high.

According to another embodiment of the present disclosure, the temperature of the 3-1 stream may be about 10 to about 300°C, preferably about 10°C or more, or about 15°C or more, or about 50 °C or more, and about 300°C or less, or about 250°C or less, or about 200°C or less.

Further, the temperature of the 3-2 stream may be about 200 to about 600°C, preferably about 250°C or more, or about 300°C or more, or about 350°C or more, or about 400 °C or more, and about 600°C or less, or about 550°C or less, or about 530°C or less.

At this time, the temperature difference between the 3-2 stream and the 3-1 stream may be preferably about 200°C or more, or about 250°C or more, and about 500°C or less, or about 450°C or less.

According to another embodiment of the present disclosure, the 3-1 stream may be sprayed at a flow rate of 0.05 g/min to 1.5 g/min, the lower limit thereof be preferably about 0.05 g/min or more, or about 0.1 g/min or more, or about 0.15 g/min or more, or about 0.18 g/min or more, and the upper limit thereof may be preferably about 1.5 g/min or less, or about 1.0 g/min or less, or about 0.8 g/min or less.

Further, the 3-2 stream may be sprayed at a flow rate of 0.1 g/min to 4.0 g/min, the lower limit thereof may be preferably about 0.1 g/min or more, or about 0.2 g/min or more, or about 0.3 g/min or more, and the upper limit thereof may be preferably about 4.0 g/min or less, or about 3.0 g/min or less, or about 2.0 g/min or less.

At this time, in the mixing and spraying step, preferably, the 3-1 stream flow rate: the 3-2 stream flow rate = 1: 1.5 to 1: 5.

On the other hand, provided herein is a method for preparing acrylic acid, comprising the steps of: obtaining lactic acid molecules by vaporizing lactic acid according to any one of the above-mentioned methods; subjecting the lactic acid molecules to a dehydration reaction to prepare acrylic acid; and obtaining the acrylic acid.

On the other hand, provided herein is an apparatus for vaporizing lactic acid, comprising: a feed supply section 100 that supplies a 3-1 stream 1 containing a lactic acid aqueous solution; a steam supply section 200 that supplies a 3-2 stream 2 containing a steam; a vaporization reaction section 300 that receives supply of a 3-1 stream from the feed supply section and receives supply of a 3-2 stream from the steam supply section to perform a vaporization reaction of a lactic acid aqueous solution; an obtaining section 400 that obtains a 3-3 stream 3 containing vaporized lactic acid molecules.

The vaporization reaction section 300 may include a spray section 310 that sprays a 3-1 stream supplied from the feed supply section and a 3-2 stream supplied from the steam supply section into the interior of the vaporization reaction section at the lower end.

The feed supply section may include a lactic acid aqueous solution feed 110 that supplies a lactic acid aqueous solution; and a feed pretreatment section 120 that adjusts the temperature and pressure of the 3-1 stream.

At this time, the feed pretreatment section 120 can discharge the 3-1 stream by adjusting the temperature to about 10 to about 300°C.

Further, the steam supply section may include a water supply section 210 that supplies water; and a water pretreatment section 220 that adjusts the temperature and pressure of the 3-2 stream.

At this time, the water pretreatment section 220 can discharge the 3-2 stream by adjusting the temperature to 200 to 600°C.

Further, the spray section may include a mix spray nozzle that mixes and sprays the 3-1 stream and the 3-2 stream.

According to another embodiment, the spray section may include a 3-1 nozzle that sprays the 3-1 stream, and a 3-2 nozzle that sprays the 3-2 stream.

The spray section can be adjusted so that the ratio of the 3-1 stream spray flow rate to the 3-2 stream spray flow rate is about 1: 1.5 to about 1: 5.

The obtaining section may be located above the vaporization reaction section.

Further, the obtaining section may include a gas-liquid separator 410 that separates and discharges vaporized lactic acid molecules and liquefied aqueous solution components.

At this time, the aqueous solution component discharged from the gas-liquid separator can be recovered and reused as a lactic acid aqueous solution feed 110.

In the present disclosure, the terms "1-1," "1-2," etc. are used to explain various elements, and these terms are only used to distinguish one constitutional element from the other constitutional elements.

The technical terms used herein is for the purpose of describing exemplary embodiments only and is not intended to limit the scope of the invention.

The singular forms "a," "an" and "the" are intended to include plural forms, unless the context clearly indicates otherwise.

It should be understood that the terms "comprise," "include", "have", etc. are used herein to specify the presence of stated features, integers, steps, components or combinations thereof, but do not preclude the presence or addition of one or more other features, integers, steps, components, or combinations thereof.

Also, as used herein, in case a layer or an element is mentioned to be formed "on" layers or elements, it means that the layer or element is directly formed on the layers or elements, or it means that other layers or elements may be additionally formed between the layers, on a subject, or on a substrate.

Although the present disclosure may have various forms and various modifications can be made thereto, specific examples will be exemplified and explained in detail. However, it is not intended to limit the present disclosure to disclosed forms, and it should be understood that all the modifications, equivalents or substitutions within the idea and technical scope of the present disclosure are included in the present disclosure.

As used herein, the term "lactic acid" is a compound represented by the following Chemical Formula, and is used as a concept encompassing all of lactic acid isomers, naturally occurring lactic acid dimers and lactic acid oligomers, unless otherwise specified herein.

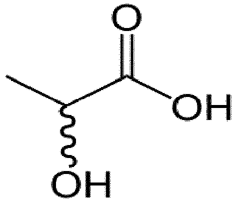

Next, the present disclosure will be described in detail.

According to an aspect of the present disclosure, there is provided a method for vaporizing lactic acid comprising the steps of: heating and pressurizing a lactic acid aqueous solution having a 1-1 concentration; spraying a 1-1 stream of a liquid phase containing the heated and pressurized lactic acid aqueous solution having a 1-1 concentration; vaporizing the lactic acid contained in the 1-1 stream through spraying; and obtaining a 1-3 stream of a gas phase containing lactic acid molecule.

Further, the method for vaporizing lactic acid can be realized by the following apparatus.

According to another aspect of the present disclosure, there is provided an apparatus for vaporizing lactic acid, comprising: a pretreatment section that heats and pressurizes a lactic acid aqueous solution having a 1-1 concentration; a feed supply section that receives the supply of the lactic acid aqueous solution from the pretreatment section and supplies a 1-1 stream containing the lactic acid aqueous solution; a spray section that sprays the 1-1 stream transferred from the feed supply section into the vaporization reactor; a vaporization reactor that vaporizes the sprayed lactic acid aqueous solution; and an obtaining section that obtains a 1-3 stream containing vaporized lactic acid molecules.

The present inventors have found that when a lactic acid aqueous solution concentrated to a high concentration is heated and pressurized to make a high temperature and high pressure state, which is sprayed into a vaporization reactor under normal pressure conditions and subjected to an instantaneous vaporization, the concentration of oligomers of lactic acid can be efficiently reduced within a very fast period of time, thereby completing the present disclosure.

Lactic acid is often used in the production of acrylic acid. When lactic acid is dehydrated to produce acrylic acid, it proceeds by a gas phase reaction and thus, lactic acid needs to be vaporized to lactic acid molecules.

However, as described above, lactic acid is usually concentrated and stored at a high concentration during storage or distribution process after production. Due to the structural feature peculiar to lactic acid molecules containing both hydroxy groups and carboxyl groups in the molecule, it often forms a dimer structure, or forms an oligomer having a form in which water molecules are removed and subjected to dehydration condensation.

Such lactic acid oligomer molecules may be carbonized in the vaporization step or reaction step to form coke, and thus reduce the active area of the reaction catalyst, and can also be included as a by-product in the final product. Above all, due to the presence of lactic acid oligomers, the content of lactic acid in the form of single molecule that can participate in the reaction is greatly reduced, it is necessary to convert the lactic acid oligomer into a single molecule form within the concentrated lactic acid aqueous solution, reduce the content of the oligomer and increase the content of lactic acid single molecule.

However, when the concentration is diluted by simply adding water to the concentrated lactic acid aqueous solution containing a high content of lactic acid oligomers, the equilibrium movement velocity is very slow and thus, a very long time is consumed to reduce the content of the lactic acid oligomer.

Generally, in order to shorten this time, a high concentration of lactic acid aqueous solution is heated and vaporized. However, since the vaporization efficiency of lactic acid is lower than that of water, the water is vaporized first, and the proportion of vaporized lactic acid is less than about 20 wt.%. In this case, as the water vaporizes first, the concentration of the remaining lactic acid aqueous solution becomes higher, which causes a problem that the concentration of the oligomer in the remaining lactic acid aqueous solution is also further increased.

Therefore, in the method of vaporizing lactic acid according to an embodiment of the present disclosure, the lactic acid aqueous solution having a 1-1 concentration is first heated and pressurized to make a high-temperature and high-pressure state, which is sprayed into a vaporization reactor. The lactic acid contained in the 1-1 stream can be instantaneously vaporized through spraying, and the 1-3 stream of the gas phase containing vaporized lactic acid molecules can be obtained.

That is, according to one embodiment of the present disclosure, rather than directly heating and vaporizing a high-concentration lactic acid aqueous solution,
i) high-concentration lactic acid aqueous solution is again heated and pressurized under high-temperature and high-pressure conditions to reach the conditions for spraying, thereby preventing a phenomenon where water is first vaporized in lactic acid aqueous solution,
ii) By supplying a lactic acid aqueous solution in a high temperature and high pressure state, it is sprayed in the form of aerosol or droplet in the vaporization reactor,
iii) High-concentration lactic acid sprayed in the form of aerosol in the vaporization reactor is instantaneously vaporized, and vaporized lactic acid single molecule can be obtained.

On the other hand, the method for vaporizing lactic acid as described above may be realized by an apparatus described later.

Such an apparatus may include a pretreatment section that heats and pressurizes a lactic acid aqueous solution having a 1-1 concentration; a feed supply section that receives the supply of the lactic acid aqueous solution from the pretreatment section and supplies a 1-1 stream containing the lactic acid aqueous solution; a spray section that sprays the 1-1 stream transferred from the feed supply section into the vaporization reactor; a vaporization reactor that vaporizes the sprayed lactic acid aqueous solution; and an obtaining section that obtains a 1-3 stream containing vaporized lactic acid molecules.

The concentration of lactic acid in the 1-3 stream may be about 30 wt.% or less, or about 25 wt.% or less, or about 20 wt.% or less, and the lower limit thereof may not have a great deal of meaning, depending on the process conditions, but may be about 0.1 wt.% or more, or about 5 wt.% or more.

Here, the 1-3 streams containing a lactic acid single molecule may more specifically contain, for example, the above-mentioned lactic acid oligomer in an amount of less than about 1 wt.%, preferably less than about 0.5 wt.%, or less than about 0.1 wt.%. More preferably, it may be desirable to be substantially free of lactic acid oligomers.

Substantially free of lactic acid oligomer means that the content of lactic acid oligomer is 0 wt.% within a detectable limit in the process.

According to an embodiment of the present disclosure, the 1-1 concentration may be about 20 to about 99 wt.%, about 20 wt.% or more, or about 30 wt.% or more, or about 40 wt.% or more, or about 50 wt.% or more, or about 60 wt.% or more, or about 70 wt.% or more, or about 75 wt.% or more, and about 99 wt.% or less, about 95 wt.% or less, or about 90 wt.% or less, or about 85 wt.% or less. That is, the lactic acid aqueous solution may be concentrated to a high concentration.

This generally corresponds to the general concentrations of commercially available lactic acid raw materials for the production, storage, distribution, and sale of lactic acid.

Here, the lactic acid aqueous solution contained in the 1-1 stream, that is, the lactic acid feed in the process, means a state in which lactic acid is dissolved in water, and as described above, this means a state that naturally contains all lactic acid single molecules, lactic acid dimers, and lactic acid oligomers depending on the temperature and concentration conditions.

Further, the lactic acid concentration described here also means the concentration of lactic acid-based compounds containing lactic acid single molecule, lactic acid dimer, and lactic acid oligomer.

More specifically, for example, when calculated by computer modeling, the oligomer content for each concentration of the total lactic acid-based compound is shown in Table 1 below.

However, this may appear a slight calculation error depending on the actual calculation model. In the actual measured value, a slight measurement error may appear depending on the measurement conditions or the measurement method (titration method or HPLC).

**[Table 1]**

| Concentrat ion of total lactic acid based compound (wt.%) | Concentrat ion of single molecule (wt.%) | Concentrat ion of dimer (wt.%) | Concentrat ion of trimer or higher (wt.%) |
|---|---|---|---|
| 5 | 5.0 | 0.019 | 0.001 |
| 10 | 9.9 | 0.079 | 0.011 |
| 15 | 14.8 | 0.187 | 0.013 |
| 20 | 19.6 | 0.35 | 0.05 |
| 25 | 24.3 | 0.575 | 0.125 |
| 30 | 29.0 | 0.874 | 0.126 |
| 35 | 33.6 | 1.26 | 0.14 |
| 40 | 38.0 | 1.75 | 0.25 |
| 45 | 42.3 | 2.35 | 0.35 |
| 50 | 46.3 | 3.11 | 0.59 |
| 55 | 50.2 | 4.03 | 0.77 |
| 60 | 53.8 | 5.18 | 1.02 |
| 65 | 56.9 | 6.58 | 1.52 |
| 70 | 59.6 | 8.31 | 2.09 |
| 75 | 61.5 | 10.4 | 3.1 |
| 80 | 62.5 | 13.0 | 4.5 |
| 85 | 62.2 | 16.2 | 6.6 |
| 90 | 60.1 | 19.8 | 10.1 |
| 95 | 55.4 | 23.6 | 16 |
| 100 | 47.6 | 26.6 | 25.8 |

According to an embodiment of the present disclosure, in the heating and pressurizing step, a lactic acid aqueous solution having the 1-1 concentration can be heated and pressurized under the conditions of a temperature of about 150°C to about 250°C, preferably about 150°C or more, or about 160°C or more, and about 250°C or less, or about 200°C or less; and a pressure of about 1 bar to about 40 bar, preferably more than about 1 bar, or about 5 bar or more, about 40 bar or less, or about 30 bar or less, or about 15 bar or less.

When the heating and pressurization step proceed to excessively low temperature and pressure outside the above temperature and pressure range, there may be a problem that the temperature of the mixture is low, and thus lactic acid does not vaporize sufficiently during spraying. When the step proceeds to excessively high temperature and pressure, there may be a problem that the pressure for maintaining the temperature is excessively high or the temperature of vaporized lactic acid is too high, and thus, lactic acid molecules are converted into other substances.

From this point of view, the pretreatment section may further include a temperature adjusting part and a pressure regulating part that heat and pressurize the lactic acid aqueous solution having the 1-1 concentration under conditions of a temperature of 150°C to 250°C and a pressure of 1 bar to 40 bar.

According to another embodiment of the present disclosure, the temperature of the 1-1 stream, that is, the temperature of the sprayed 1-1 stream may be about 10 to about 300°C, preferably about 10°C or more, or about 50°C or more, or about 150°C or more, and about 300°C or less, or about 250°C or less, or about 200°C or less.

When the 1-1 stream has a temperature that is too low outside the temperature range, there may be a problem that some of the vaporized lactic acid is condensed, and when the temperature is too high, there may be a problem that lactic acid molecules are converted into other substances.

From this point of view, the feed supply section may further include a feed temperature adjusting part that adjusts the temperature of the 1-1 stream.

The lactic acid aqueous solution supplied from the feed is heated and then sprayed in the form of droplets into the vaporization reactor via a transfer line and a nozzle.

That is, according to another embodiment of the present disclosure, the spray section may include a 1-1 stream nozzle that sprays the 1-1 stream into the vaporization reactor.

According to another embodiment of the present disclosure, the 1-1 stream can be preferably sprayed at a flow rate of about 0.1 g/min to 1.0 g/min, preferably about 0.1 g/min or more, or about 0.15 g/min or more, about 1.0 g/min or less, or about 0.5 g/min or less, or about 0.3 g/min or less.

When the spray amount and spray rate of the 1-1 stream are too low outside the above range, there may be a problem that the flow rate of the supplied lactic acid is too low and thus, overreaction proceeds in the dehydration reaction proceeding to the next step. When the spray amount and spray rate are too high, there may be a problem that the amount of heat required for vaporization increases which may lead to incomplete vaporization.

Further, at this time, separately from the 1-1 stream, the 1-2 stream including high-temperature steam may be sprayed into the vaporization reactor via a transfer line and nozzle separate from the 1-1 stream. That is, it may be preferable that the 1-1 stream and the 1-2 stream are mixed and sprayed together into a vaporization reactor.

From this point of view, according to another embodiment of the present disclosure, the apparatus for vaporizing lactic acid further comprises a steam supply section that supplies a steam, and the spray section may further include a 1-2 stream nozzle that receives supply of a steam from the steam generator and sprays a 1-2 stream of a gas phase containing a steam.

In this case, the high-temperature steam supplied to the 1-2 stream can be mixed with the lactic acid supplied to the 1-1 stream to instantly dilute the lactic acid to a lower concentration. Furthermore, heat energy can be transferred to the lactic acid molecule, the oligomer content in the lactic acid can be rapidly reduced, thus assisting in the vaporization of the lactic acid molecule.

At the time of mixing and spraying, the transfer lines of the 1-1 stream and the 1-2 stream are first combined before spraying, and then mixed and sprayed through the same nozzle, but more preferably, the 1-1 stream and the 1-2 stream are mixed and sprayed into the vaporization reactor through separate nozzles, respectively.

From this point of view, the temperature of the 1-2 streams may be about 200 to about 600°C, preferably about 250°C or more, or about 300°C or more, or about 350 °C or more, or about 400°C or more, and about 600°C or less, or about 550°C or less, or about 530 °C or less.

At this time, the temperature difference between the 1-2 stream and the 1-1 stream may be preferably about 200 °C or more, or about 250 °C or more, and about 500°C or less, or about 450°C or less.

When the temperature of the 1-2 stream is too low outside the above range, there may be a problem that the temperature of the mixture is too low and thus, the lactic acid is not sufficiently vaporized. When the temperature of the 1-2 stream is too high outside the above range, there may be a problem that the pressure is excessively increased to maintain the temperature.

From this point of view, the steam supply section may further include a steam temperature adjusting part that adjusts the temperature of the 1-2 stream.

Further, the 1-2 stream can be preferably sprayed at a flow rate of 0.1 g/min to 3.0 g/min, preferably about 0.1 g/min or more, or about 0.2 g/min or more, or about 0.3 g/min or more, and about 3.0 g/min or less, or about 2.0 g/min or less, or about 1.0 g/min or less.

When the spray amount and spray rate of the 1-2 stream are too low outside the above ranges, there may be a problem that the heat supplied for vaporization decreases which may lead to incomplete vaporization. When the spray amount and spray rate are too high, the concentration of lactic acid is diluted, which may cause problems in the subsequent separation of the product.

At this time, in the mixing and spraying step, the flow rate of the 1-1 stream: the flow rate of the 1-2 stream may be preferably about 1: 1.5 to about 1: 5.

According to another embodiment of the present disclosure, before the 1-1 stream and the 1-2 stream are mixed and sprayed in the form of droplets in the vaporization reactor via a transfer line and a nozzle, the inside of the vaporization reactor is preferably saturated with steam in advance.

By this method, when the 1-1 stream is sprayed into the reactor under high temperature conditions, water evaporates in the droplets of the 1-1 stream instantaneously sprayed under high temperature conditions, thereby preventing lactic acid from being concentrated.

At this time, it may be preferable that the vaporization reactor proceeds the reaction while maintaining a temperature condition of about 300 to about 400°C.

On the other hand, provided herein is a method for preparing acrylic acid, comprising the steps of: obtaining lactic acid molecules by vaporizing lactic acid according to any one of the above-mentioned methods; subjecting the lactic acid molecules to a dehydration reaction to prepare acrylic acid; and obtaining the acrylic acid.

The reaction of producing acrylic acid by intramolecular dehydration of lactic acid can be represented by the following Chemical Formula, which is known to proceed in one step in the presence of a catalyst.

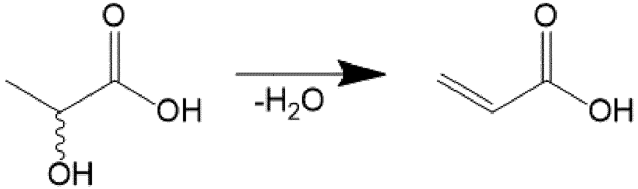

However, in the case of the method for preparing acrylic acid according to one aspect of the present disclosure, except for the content related to vaporization of lactic acid described above, a catalyst, a reactor, or reaction conditions generally known in the art can be employed for the method for producing acrylic acid from vaporized lactic acid.

For example, the catalyst used for the dehydration reaction of lactic acid may include CaSO₄/ Na₂SO₄; Na₄P₂O₇/CaSO₄; Na₄P₂O₇/Ca₃(PO₄)₂; NaH₂PO₄-NaHCO₃/SiO₂; AlPO₄-NH₃; Ca₃(PO₄)₂/CaSO₄ and the like.

The dehydration reaction using a solid catalyst may proceed by a continuous reaction using a fixed reactor, a batch reaction, or the like. When a fixed reactor is used, the product can be continuously produced by charging a solid catalyst in the reactor and continuously supplying the reactants to the reactor for reaction.

The temperature of the dehydration reaction may be about 300 to about 500 °C, or about 350 to about 450°C. The reaction pressure may be from about 1 atmosphere to about 5 atmospheres, or from about 1 atmosphere to about 2 atmospheres.

The supply velocity of the reactants may vary depending on the shape or type of reactor, other reaction conditions, etc. Specifically, for example, the gas phase lactic acid supply velocity (Weight Hourly Space Velocity, WHSV) may be advanced to be about 0.05 to about 1.0 / hr, or about 0.10 to about 0.50 / hr.

If the supply velocity deviates from the above conditions, the decomposition reaction by hydrogenation proceeds, which may cause a problem that the reaction efficiency is lowered or the conversion rate is lowered.

On the other hand, according to another aspect of the present disclosure, there is provided a method for vaporizing lactic acid comprising the steps of: mixing and spraying a 3-1 stream of a liquid phase containing a lactic acid aqueous solution and a 3-2 stream of a gas phase containing a steam; vaporizing the lactic acid aqueous solution through heat exchange between the 3-1 stream and the 3-2 stream; and obtaining a 3-3 stream of a gas phase containing lactic acid single-molecule.

According to yet another aspect of the present disclosure, there is provided an apparatus for vaporizing lactic acid, comprising: a feed supply section 100 that supplies a 3-1 stream 1 containing a lactic acid aqueous solution; a steam supply section 200 that supplies a 3-2 stream 2 containing a steam; a vaporization reaction section 300 that receives a supply of a 3-1 stream from the feed supply section and receives a supply of a 3-2 stream from the steam supply section to perform a vaporization reaction of a lactic acid aqueous solution; and an obtaining section 400 that obtains a 3-3 stream 3 containing vaporized lactic acid molecules.

In the case of using the above apparatus, a method for vaporizing lactic acid can be realized comprising the steps of: mixing and spraying a 3-1 stream of a liquid phase containing a lactic acid aqueous solution and a 3-2 stream of a gas phase containing a steam; vaporizing the lactic acid aqueous solution through heat exchange between the 3-1 stream and the 3-2 stream; and obtaining a 3-3 stream of a gas phase containing lactic acid single-molecule.

The present inventors have found that when a lactic acid aqueous solution concentrated to a high concentration is mixed with a steam and vaporized through direct heat exchange, the concentration of oligomers of lactic acid can be efficiently reduced within a very fast time, thereby completing the present disclosure.

Lactic acid is often used in the production of acrylic acid. When lactic acid is dehydrated to produce acrylic acid, it proceeds by a gas phase reaction and thus, lactic acid needs to be vaporized to lactic acid molecules.

However, as described above, lactic acid is usually concentrated and stored at a high concentration during storage or distribution process after production. Due to the structural feature peculiar to lactic acid molecules containing both hydroxy groups and carboxyl groups in the molecule, it often forms a dimer structure or is dehydrated and condensed to form an oligomer in which water molecules are removed.

Such lactic acid oligomer molecules may be carbonized in the vaporization step or reaction step to form coke, and thus reduce the active area of the reaction catalyst, and can also be included as a by-product in the final product. Above all, due to the presence of lactic acid oligomers, the content of lactic acid in the form of single molecule that can participate in the reaction is greatly reduced, it is necessary to convert the lactic acid oligomer into a single molecule form in the concentrated lactic acid aqueous solution, reduce the content of the oligomer and increase the content of lactic acid single molecule.

However, when the concentration is diluted by simply adding water to the concentrated lactic acid aqueous solution containing a high content of lactic acid oligomers, the equilibrium movement velocity is very slow and thus, a very long time is consumed to reduce the content of the lactic acid oligomer.

Generally, in order to shorten this time, a 80 wt.% or higher concentration of lactic acid aqueous solution is heated and vaporized. However, since the vaporization efficiency of lactic acid is lower than that of water, the water is vaporized first, and the proportion of vaporized lactic acid is less than about 20 wt.%. In this case, as the water vaporizes first, the concentration of the remaining lactic acid aqueous solution becomes higher, which causes a problem that the concentration of the oligomer in the remaining lactic acid aqueous solution is further increased.

Therefore, the method of vaporizing lactic acid according to an embodiment of the present disclosure comprises the steps of: mixing and spraying a 3-1 stream of a liquid phase containing a lactic acid aqueous solution and a 3-2 stream of a gas phase containing a steam; vaporizing the lactic acid aqueous solution through heat exchange between the 3-1 stream and the 3-2 stream; and obtaining a 3-3 stream of a gas phase containing lactic acid single-molecule.

Further, the apparatus for vaporizing lactic acid according to an aspect of the present disclosure comprises a feed supply section 100 that supplies a 3-1 stream 1 containing a lactic acid aqueous solution; a steam supply section 200 that supplies a 3-2 stream 2 containing a steam; a vaporization reaction section 300 that receives a supply of a 3-1 stream from the feed supply section and receives a supply of a 3-2 stream from the steam supply section to perform a vaporization reaction of a lactic acid aqueous solution; an obtaining section 400 that obtains a 3-3 stream 3 containing vaporized lactic acid molecules.

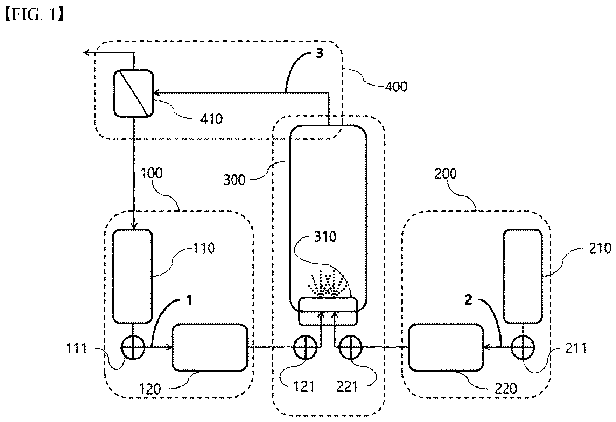

Fig. 1 is a schematic diagram showing an apparatus for vaporizing lactic acid according to an embodiment of the present disclosure.

Referring to Fig. 1, an embodiment of the apparatus for vaporizing lactic acid can be confirmed, comprising: a feed supply section 100 that supplies a 3-1 stream 1 containing a lactic acid aqueous solution; a steam supply section 200 that supplies a 3-2 stream 2 containing steam; a vaporization reaction section 300 that receives a supply of a 3-1 stream from the feed supply section and receives a supply of a 3-2 stream from the steam supply section to perform a vaporization reaction of a lactic acid aqueous solution; and an obtaining section 400 that obtains a 3-3 stream 3 containing vaporized lactic acid molecules.

That is, according to one embodiment of the present disclosure, rather than directly heating and vaporizing a high-concentration lactic acid aqueous solution, a high-concentration lactic acid aqueous solution is sprayed in a kind of aerosol form, but high-temperature steam is mixed and sprayed together thereto, so that the concentration of lactic acid is reduced, and at the same time, lactic acid is vaporized through rapid heat exchange between the high-concentration lactic acid sprayed in the form of an aerosol and steam. Thereby, a single molecule of vaporized lactic acid can be obtained.

The concentration of lactic acid in the 3-3 stream may be about 30 wt.% or less, or about 25 wt.% or less, or about 20 wt.% or less, and the lower limit thereof may not have a great deal of meaning depending on process conditions, but may be about 0.1 wt.% or more, or about 5 wt.% or more.

Here, the 3-3 streams containing a lactic acid single molecule more specifically contain, for example, the above-mentioned lactic acid oligomer in an amount of less than about 1 wt.%, preferably less than about 0.5 wt.%, or less than about 0.1 wt.%. More preferably, it may be desirable to be substantially free of lactic acid oligomers.

Substantially free of lactic acid oligomer means that the content of lactic acid oligomer is 0 wt.% within a detectable limit in the process.

According to an embodiment of the present disclosure, the lactic acid aqueous solution contained in the 3-1 stream may be concentrated to a high concentration so that the lactic acid concentration is about 40 to about 99 wt.%, about 45 wt.% or more, or about 50 wt.% or more, or about 60 wt.% or more, or about 70 wt.% or more, or about 75 wt.% or more, and about 99 wt.% or less, about 95 wt.% or less, or about 90 wt.% or less, or about 85 wt.% or less.

Further, the concentration of a multimer in the lactic acid aqueous solution contained in the 3-1 stream, that is, the concentration of the lactic acid dimer or the lactic acid oligomer of trimer or higher may be about 2 to about 55 wt.%, or about 2 wt.% or more, or about 5 wt.% or more, or about 7 wt.% or more, or about 8 wt.% or more, and about 55 wt.% or less, or about 40 wt.% or less, or about 20 wt.% or less. The content of the multimer may be relatively high.

Here, the lactic acid aqueous solution contained in the 3-1 stream, that is, the lactic acid feed in the process, means a state in which lactic acid is dissolved in water, and as described above, this means a state that naturally contains all lactic acid single molecules, lactic acid dimers, and lactic acid oligomers depending on the temperature and concentration conditions. Further, the lactic acid concentration described here also means the concentration of lactic acid-based compounds containing lactic acid single molecule, lactic acid dimer, and lactic acid oligomer.

More specifically, for example, when calculated by computer modeling based on the known oligomerization equilibrium constant of lactic acid, the oligomer content for each concentration of the total lactic acid-based compound is shown in Table 2 below.

However, this may appear a slight calculation error depending on the actual calculation model, and even in the actual measured value, a slight measurement error may appear depending on the measurement conditions or the measurement method (titration method or HPLC).

**[Table 2]**

| Concentrat ion of total lactic acid-based compound s (wt.%) | Concentrat ion of single molecule (wt.%) | Concentrat ion of dimer (wt.%) | Concentrat ion of trimer or higher (wt.%) |
|---|---|---|---|
| 5 | 5.0 | 0.019 | 0.001 |
| 10 | 9.9 | 0.079 | 0.011 |
| 15 | 14.8 | 0.187 | 0.013 |
| 20 | 19.6 | 0.35 | 0.05 |
| 25 | 24.3 | 0.575 | 0.125 |
| 30 | 29.0 | 0.874 | 0.126 |
| 35 | 33.6 | 1.26 | 0.14 |
| 40 | 38.0 | 1.75 | 0.25 |
| 45 | 42.3 | 2.35 | 0.35 |
| 50 | 46.3 | 3.11 | 0.59 |
| 55 | 50.2 | 4.03 | 0.77 |
| 60 | 53.8 | 5.18 | 1.02 |
| 65 | 56.9 | 6.58 | 1.52 |
| 70 | 59.6 | 8.31 | 2.09 |
| 75 | 61.5 | 10.4 | 3.1 |
| 80 | 62.5 | 13.0 | 4.5 |
| 85 | 62.2 | 16.2 | 6.6 |
| 90 | 60.1 | 19.8 | 10.1 |
| 95 | 55.4 | 23.6 | 16 |
| 100 | 47.6 | 26.6 | 25.8 |

Further, the lactic acid aqueous solution (3-1 stream) supplied from the feed may be subjected to a heating process.

For this purpose, the feed supply section may include a lactic acid aqueous solution feed 110 that supplies the lactic acid aqueous solution; and a feed pretreatment section 120 that adjusts the temperature and pressure of the 3-1 stream.

At this time, the feed pretreatment section 120 may discharge the 3-1 stream by adjusting it to a temperature of about 10 to about 300°C and a pressure of 1 to 50 atmospheres.

More specifically, the temperature of the 3-1 stream discharged from the feed pretreatment section may be about 10 to about 300°C, preferably about 10°C or more, or about 15°C or more, or about 50°C or more, and about 300°C or less, or about 250°C or less, or about 200°C or less.

When the 3-1 stream has a temperature that is too low outside the temperature range, there may be a problem that some of the vaporized lactic acid is condensed, and when the temperature is too high, there may be a problem that lactic acid molecules are converted into other substances.

The lactic acid aqueous solution supplied from the feed is heated and then sprayed in the form of droplets into the vaporization reactor via a transfer line and a nozzle. The temperature of the lactic acid aqueous solution is kept high, and as the pressure is higher, it is more advantageous to obtain small-sized droplets.

At this time, separately from the 3-1 stream, the 3-2 stream containing a steam is also mixed and sprayed with the 3-1 stream in the form of droplets in the vaporization reactor via a transfer line and a nozzle.

At the time of mixing and spraying, the transfer lines of the 3-1 stream and the 3-2 stream are first combined before spraying, and then mixed and sprayed through the same nozzle, but more preferably, the 3-1 stream and the 3-2 stream are mixed and sprayed into the vaporization reactor via separate nozzles.

For this purpose, the vaporization reaction section 300 may include a spray section 310 that sprays a 3-1 stream supplied from the feed supply section and a 3-2 stream supplied from the steam supply section at the lower end into the interior of the vaporization reaction section.

In the spray section, the 3-1 stream and the 3-2 stream may be i) combined in a step before spraying, and mixed and sprayed into the vaporization reaction section, or ii) combined into a single nozzle in the spraying step, mixed and sprayed into the vaporization reaction section, or iii) mixed and sprayed into the vaporization reaction section through a separate nozzle.

That is, the spray section may include a mix spray nozzle that mixes and sprays the 3-1 stream and the 3-2 stream.

According to another example, the spray section may include a 3-1 nozzle that sprays the 3-1 stream, and a 3-2 nozzle that sprays the 3-2 stream.

However, in order to prevent the concentration of the lactic acid aqueous solution from lowering during the transfer process, it is more preferable to mix and spray the 3-1 stream and the 3-2 stream into the vaporization reactor via separate nozzles.

The 3-2 stream instantaneously lowers the concentration of lactic acid in the 3-1 stream containing highly concentrated lactic acid, and at the same time, transfers thermal energy to the 3-1 stream, promote vaporization of lactic acid molecules and thus reduce the ratio of oligomers.

From this point of view, the temperature of the 3-2 stream may be about 200 to about 600°C, preferably about 250°C or more, or about 300°C or more, or about 350°C or more, or about 400 °C or more, and about 600°C or less, or about 550°C or less, or about 530°C or less.

For this purpose, the steam supply section may include a water supply section 210 that supplies water; and a water pretreatment section 220 that adjusts the temperature and pressure of the 3-2 stream.

Further, the water pretreatment section 220 may adjust the 3-2 stream to the above-mentioned temperature and a pressure range of about 1 to 10 atmospheres, and discharge it.

The temperature difference between the 3-2 stream and the 3-1 stream may be preferably about 200 °C or more, or about 250°C or more, and about 500°C or less, or about 450°C or less.

When the temperature of the 3-2 stream is too low outside the above range, there may be a problem that the temperature of the mixture is low and lactic acid does not vaporize sufficiently. When the temperature of the 3-2 stream is too high outside the above range, there may be a problem that the pressure is excessively increased to maintain the temperature.

According to another embodiment of the present disclosure, the 3-1 stream may be sprayed at a flow rate of 0.05 g/min to 1.5 g/min, the lower limit thereof may be preferably about 0.05 g/min or more, or about 0.1 g/min or more, or about 0.15 g/min or more, or about 0.18 g/min or more, and the upper limit thereof may be preferably about 1.5 g/min or less, or about 1.0 g/min or less, or about 0.8 g/min or less.

When the spray amount and spray rate of the 3-1 stream are too low outside the above ranges, there may be problem that the flow rate of the supplied lactic acid is too low and thus, overreaction proceeds in the dehydration reaction proceeding to the next step. When the spray amount and spray rate are too high, the amount of heat required for vaporization may be high, which may lead to incomplete vaporization.

Further, the 3-2 stream may be sprayed at a flow rate of 0.1 g/min to 4.0 g/min, the lower limit thereof may be preferably about 0.1 g/min or more, or about 0.2 g/min or more, or about 0.3 g/min or more, and the upper limit thereof may be preferably about 4.0 g/min or less, or about 3.0 g/min or less, or about 2.0 g/min or less.

When the spray amount and the spray rate of the 3-2 stream are too low outside the above ranges, less heat is supplied to the vaporization, which may lead to incomplete vaporization. When the spray amount and the spray rate are too high, the concentration of lactic acid is diluted, which may cause problems in the subsequent product separation process.

In the mixing and spraying step, the 3-1 stream flow rate: the 3-2 stream flow rate may be preferably about 1: 1.5 to about 1: 5.

When the flow rate ratio of the 3-1 stream is too small outside the above range, there may be a problem that the flow rate of the supplied lactic acid is small, so the efficiency may be lowered, and the load increases in the product separation process. When the flow rate ratio of the 3-2 stream is too small, less heat is supplied to the vaporization, which may lead to incomplete vaporization.

According to another embodiment of the present disclosure, before the 3-1 stream and the 3-2 stream are mixed and sprayed in the form of droplets in the vaporization reactor via a transfer line and a nozzle, the inside of the vaporization reactor is preferably saturated with steam in advance.

By this method, when the 3-1 stream is sprayed into the reactor under high temperature conditions, water evaporates within the droplets of the 3-1 stream instantaneously sprayed under high temperature conditions, thereby preventing lactic acid from being concentrated.

At this time, it may be preferable that the vaporization reactor proceeds the reaction while maintaining a temperature condition of about 150 to about 250°C.

The obtaining section may be located above the vaporization reaction section.

After the 3-1 stream and the 3-2 stream supplied to the lower part of the vaporization reaction section are mixed and sprayed, the lactic acid aqueous solution contained in the 3-1 stream is instantaneously vaporized, and the produced lactic acid single molecules move to the obtaining section at the top of the vaporization reaction section.

The obtaining section can include a gas-liquid separator 410 that separates and discharges vaporized lactic acid molecules and liquefied aqueous solution components.

At this time, the aqueous solution component discharged from the gas-liquid separator may be recovered and reused as the lactic acid aqueous solution feed 110.

On the other hand, provided herein is a method for preparing acrylic acid, comprising the steps of: obtaining lactic acid molecules by vaporizing lactic acid according to any one of the above-mentioned methods; subjecting the lactic acid molecules to a dehydration reaction to prepare acrylic acid; and obtaining the acrylic acid.

The reaction of producing acrylic acid by intramolecular dehydration of lactic acid can be represented by the following Chemical Formula, which is known to proceed in one step in the presence of a catalyst.

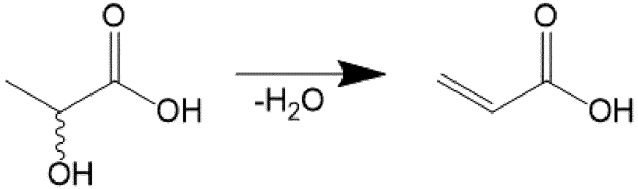

However, in the case of the method for preparing acrylic acid according to one aspect of the present disclosure, except for the content related to vaporization of lactic acid described above, a catalyst, a reactor, or reaction conditions generally known in the art can be employed for the method for producing acrylic acid from vaporized lactic acid.

For example, the catalyst used for the dehydration reaction of lactic acid may include CaSO₄/ Na₂SO₄; Na₄P₂O₇/CaSO₄; Na₄P₂O₇/Ca₃(PO₄)₂; NaH₂PO₄-NaHCO₃/SiO₂; AlPO₄-NH₃; Ca₃(PO₄)₂/CaSO₄ and the like.

The dehydration reaction using a solid catalyst may proceed by a continuous reaction using a fixed reactor, a batch reaction, or the like. When a fixed reactor is used, the product can be continuously produced by charging a solid catalyst in the reactor and continuously supplying the reactants to the reactor for reaction.

The temperature of the dehydration reaction may be about 300 to about 500 °C, or about 300 to about 400°C. The reaction pressure may be from about 1 atmosphere to about 5 atmospheres, or from about 1 atmosphere to about 2 atmospheres.

The supply velocity of the reactants may vary depending on the shape or type of reactor, other reaction conditions, etc. Specifically, for example, the gas phase lactic acid supply velocity (Weight Hourly Space Velocity, WHSV) may be advanced to be about 0.05 to about 1.0 / hr, or about 0.10 to about 0.50 / hr.

If the above conditions are not met, the decomposition reaction by oligomerization or hydrogenation proceeds, which may cause a problem that the reaction efficiency is lowered or the conversion rate is lowered.

### [Advantageous Effects]

According to the method and apparatus for vaporizing lactic acid according to an aspect of the present disclosure, the content of lactic acid oligomers can be reduced and the content of lactic acid single molecule can be increased within a short period of time, in a lactic acid aqueous solution concentrated to a high concentration.

### [BRIEF DESCRIPTION OF DRAWING]

Fig. 1 is a schematic diagram showing the apparatus for vaporizing lactic acid according to an embodiment of the present disclosure.

### [DETAILED DESCRIPTION OF THE EMBODIMENTS]

Hereinafter, the action and effect of the invention will be described in more detail with reference to specific examples of the invention. However, these examples are presented for illustrative purposes only and the scope of the invention is not limited thereby in any way.

### <First Example>

A lactic acid aqueous solution having a concentration of about 80 wt.% was prepared.

In the pretreatment section, the lactic acid aqueous solution was heated and pressurized under the conditions of 180°C and 10 atm, and prepared for supplying as a feed.

**[Table 3]**

| | Concentration of lactic acid aqueous solution (wt.%) | Ratio of oligomer | Temperature (°C) | Pressure (atm) |
|---|---|---|---|---|
| Example 1 | 80 | 0.23 | 180 | 10 |
| Example 2 | 80 | 0.24 | 180 | 10 |

The concentration of lactic acid aqueous solution, that is, the lactic acid aqueous solution concentration of the feed containing all of lactic acid, lactic acid dimer and lactic acid oligomer was determined by collecting each sample, measuring the carbon content by elemental analysis, and dividing by the ratio of carbon content in lactic acid.

The ratio of lactic acid oligomer (including dimer) in the lactic acid aqueous solution was calculated by collecting each sample, analyzing the content of lactic acid single molecules through HPLC, and dividing the amount of oligomers by the amount of total lactic acid-based compounds from the concentration of the aqueous lactic acid solution and the content of single molecules.

Next, an apparatus for vaporizing lactic acid, comprising: a feed supply section that supplies a 1-1 stream containing an aqueous lactic acid solution; a steam supply section that supplies a 1-2 stream containing a steam; a spray section that receives supply of an aqueous lactic acid solution from the feed supply section and receives supply of a steam from the steam supply section and mixes and sprays through separate nozzle; a vaporization reactor that vaporizes the sprayed aqueous lactic acid solution; and an obtaining section that obtains a 1-3 stream containing vaporized lactic acid molecules, was prepared.

A thermometer and a temperature control device were provided in the feed supply section and the steam supply section, whereby the temperature of the supplied feed (1-1 stream) and water vapor (1-2 stream) can be adjusted. The obtaining section was equipped with a thermometer to measure the temperature of the 1-3 stream.

First, steam was charged into the vaporization reactor so that the interior of the vaporization reactor was saturated with steam.

Then, the lactic acid aqueous solution according to Examples 1 to 7 was supplied to the 1-1 stream through the feed supply section, the steam was supplied through the steam supply section, and continuously mixed and sprayed into the inside of the vaporization reaction section through each nozzle. Thereby, the 1-3 stream containing vaporized lactic acid molecules was obtained through the obtaining section.

The spray conditions are as summarized in Table 4 below.

**[Table 4]**

| | Temperatur e of lactic acid aqueous solution (°C) | Pressure of lactic acid aqueous solution (atm) | Spray amount of lactic acid aqueous solution (g/min) | Steam temperature (°C) | Amount of steam sprayed |
|---|---|---|---|---|---|
| Example 1 | 180 | 10 | 0.2 | 450 | 0.60 |
| Example 2 | 180 | 10 | 0.2 | 500 | 0.35 |

Streams 1-3 obtained were analyzed, and the concentration of total lactic acid compounds in the 1-3 stream and the ratio of oligomers among them were measured and calculated, which are summarized in Table 5 below.

**[Table 5]**

| | Temperat ure of the 1-3 stream (°C) | Concentrati on of lactic acid (wt.%) | Ratio of oligomer |
|---|---|---|---|
| Example 1 | 180 | 19 | 0.04 |
| Example 2 | 180 | 9 | 0.03 |

Referring to Table 5, it can be clearly confirmed that according to Examples of the present disclosure, lactic acid can be continuously vaporized to reduce the content of lactic acid oligomer in a very short period of time.

### <Second Example>

Separately from this, a lactic acid aqueous solution having the concentration as summarized in Table 6 below was prepared.

**[Table 6]**

| | Concentration of lactic acid aqueous solution (wt.%) | Ratio of initial oligomer | Temperature (°C) | Pressure (atm) |
|---|---|---|---|---|
| Example 1 | 80 | 0.24 | 25 | 1 |
| Example 2 | 80 | 0.23 | 80 | 1 |
| Example 3 | 80 | 0.24 | 80 | 1 |
| Example 4 | 70 | 0.15 | 80 | 1 |
| Example 5 | 60 | 0.1 | 80 | 1 |
| Example 6 | 80 | 0.23 | 180 | 10 |
| Example 7 | 80 | 0.24 | 180 | 10 |

The concentration of lactic acid aqueous solution, that is, the lactic acid aqueous solution concentration of the feed containing all of lactic acid, lactic acid dimer and lactic acid oligomer was determined by collecting each sample, measuring the carbon content by elemental analysis, and dividing by the ratio of carbon content in lactic acid.

The ratio of lactic acid oligomer (including dimer) in the lactic acid aqueous solution was calculated by collecting each sample, analyzing the content of lactic acid single molecules through HPLC, and dividing the amount of oligomers by the amount of total lactic acid-based compounds from the concentration of the aqueous lactic acid solution and the content of single molecules.

Next, an apparatus for vaporizing lactic acid as shown in figure 1, comprising: a feed supply section that supplies a 3-1 stream containing an aqueous lactic acid solution; a steam supply section that supplies a 3-2 stream containing a steam; a spray section that receives supply of an aqueous lactic acid solution from the feed supply section and receives supply of a steam from the steam supply section and mixes and sprays through separate nozzle; a vaporization reaction reactor that vaporizes the sprayed aqueous lactic acid solution; and an obtaining section that obtains a 3-3 stream containing vaporized lactic acid molecules, was prepared.

A thermometer and a temperature control device were provided in the feed supply section and the steam supply section, whereby the temperature of the supplied feed (3-1 stream) and water vapor (3-2 stream) can be adjusted. The obtaining section was equipped with a thermometer to measure the temperature of the 3-3 stream.

First, steam was charged into the vaporization reactor so that the interior of the vaporization reactor was saturated with steam.

Then, the lactic acid aqueous solution according to Examples 1 to 7 was supplied to the 3-1 stream through the feed supply section, the steam was supplied through the steam supply section, and continuously mixed and sprayed into the inside of the vaporization reaction section through each nozzle. Thereby, the 3-3 stream containing vaporized lactic acid molecules was obtained through the obtaining section.

The spray conditions are as summarized in Table 7 below.

**[Table 7]**

| | Temperatur e of lactic acid aqueous solution (°C) | Pressure of lactic acid aqueous solution (atm) | Spray amount of lactic acid aqueous solution (g/min) | Steam temperature (°C) | Spray amount of steam (g/min) |
|---|---|---|---|---|---|
| Example 1 | 25 | 1 | 0.6 | 450 | 1.8 |
| Example 2 | 80 | 1 | 0.6 | 450 | 1.8 |
| Example 3 | 80 | 1 | 0.4 | 500 | 1.2 |
| Example 4 | 80 | 1 | 0.4 | 500 | 1.0 |
| Example 5 | 80 | 1 | 0.2 | 500 | 0.40 |
| Example 6 | 180 | 10 | 0.2 | 450 | 0.60 |
| Example 7 | 180 | 10 | 0.2 | 500 | 0.35 |

Streams 3-3 obtained were analyzed, and the concentration of total lactic acid compounds in the 3-3 stream and the ratio of oligomers among them were measured and calculated, which are summarized in Table 8 below.

**[Table 8]**

| | Temperatur e of the 3-3 stream (°C) | Concentrati on of lactic acid (wt.%) | Ratio of oligomer |
|---|---|---|---|
| Example 1 | 25 | 14 | 0.08 |
| Example 2 | 80 | 16 | 0.10 |
| Example 3 | 80 | 17 | 0.12 |
| Example 4 | 80 | 15 | 0.06 |
| Example 5 | 80 | <1 | 0 |
| Example 6 | 180 | 19 | 0.04 |
| Example 7 | 180 | 9 | 0.03 |

Referring to Table 8, it can be clearly confirmed that according to Examples of the present disclosure, lactic acid can be continuously vaporized to reduce the content of lactic acid oligomer in a very short period of time.

### [Description of Reference Numerals]

| | | | |
|---|---|---|---|
| 1: | 3-1 stream; | 2: | 3-2 stream; |
| 3: | 3-3 stream; | 100: | feed supply section; |
| 110: | lactic acid aqueous solution feed; | 120: | feed pretreatment section; |
| 200: | steam supply section; | 210: | water supply section; |
| 220: | water pretreatment section; | 300: | vaporization reaction section;vaporization reaction section; |
| 310: | spray section; | 400: | obtaining section; |
| 410: | gas-liquid separator; | 111, 121, 211, 221: | flow regulator (valve) |

## Claims

1. A method for vaporizing lactic acid comprising the steps of:
heating and pressurizing a lactic acid aqueous solution having a 1-1 concentration;
spraying a 1-1 stream of a liquid phase containing the heated and pressurized lactic acid aqueous solution having a 1-1 concentration;
vaporizing the lactic acid contained in the 1-1 stream through spraying; and
obtaining a 1-3 stream of a gas phase containing lactic acid molecule.

2. The method for vaporizing lactic acid according to claim 1, wherein:
in the heating and pressurizing step, the lactic acid aqueous solution having the 1-1 concentration is heated and pressurized under conditions of a temperature of 150°C to 250°C and a pressure of 1 bar to 40 bar.

3. The method for vaporizing lactic acid according to claim 1, wherein:
the 1-1 concentration is 20 to 99 wt.%.

4. The method for vaporizing lactic acid according to claim 1, wherein:
a temperature of the 1-1 stream is 10°C to 300°C.

5. The method for vaporizing lactic acid according to claim 1, wherein:
the 1-1 stream is sprayed at a flow rate of 0.1 g/min to 1.0 g/min.

6. The method for vaporizing lactic acid according to claim 1, wherein:
when the 1-1 stream is sprayed, the 1-1 stream and the 1-2 stream of the gas phase containing a steam are mixed and sprayed.

7. The method for vaporizing lactic acid according to claim 6, wherein:
a temperature of the 1-2 stream is 200°C to 600°C.

8. The method for vaporizing lactic acid according to claim 6, wherein:
the 1-2 stream is sprayed at a flow rate of 0.1 g/min to 3.0 g/min.

9. The method for vaporizing lactic acid according to claim 6, wherein:
in the mixing and spraying step, the 1-1 stream flow rate: the 1-2 stream flow rate = 1: 1.5 to 1: 5.

10. A method for preparing acrylic acid, comprising the steps of:
obtaining lactic acid molecules by vaporizing lactic acid according to the method of any one of claims 1 to 9;
subjecting the lactic acid molecules to a dehydration reaction to prepare acrylic acid; and
obtaining the acrylic acid.

11. An apparatus for vaporizing lactic acid, comprising:
a pretreatment section that heats and pressurizes a lactic acid aqueous solution having a 1-1 concentration;
a feed supply section that receives the supply of the lactic acid aqueous solution from the pretreatment section and supplies a 1-1 stream containing the lactic acid aqueous solution;
a spray section that sprays the 1-1 stream transferred from the feed supply section into the vaporization reactor;
a vaporization reactor that vaporizes the sprayed lactic acid aqueous solution; and
an obtaining section that obtains a 1-3 stream containing vaporized lactic acid molecules.

12. The apparatus for vaporizing lactic acid according to claim 11, wherein:
the pretreatment section further comprises a temperature adjusting part and a pressure regulating part that heat and pressurize the lactic acid aqueous solution having the 1-1 concentration under conditions of a temperature of 150°C to 250°C and a pressure of 1 bar to 40 bar.

13. The apparatus for vaporizing lactic acid according to claim 12, wherein:
the 1-1 concentration is 20 to 99 wt.%.

14. The apparatus for vaporizing lactic acid according to claim 11, wherein:
the feed supply section further comprises a feed temperature adjusting part that adjusts the temperature of the 1-1 stream to 150 to 300°C.

15. The apparatus for vaporizing lactic acid according to claim 11, wherein:
the spray section comprises a 1-1 stream nozzle that adjusts so that the 1-1 stream is sprayed at a flow rate of 0.1 g/min to 1.0 g/min.

16. The apparatus for vaporizing lactic acid according to claim 11, wherein:
the apparatus for vaporizing lactic acid further comprises a steam supply section that supplies steam, and
the spray section further comprises a 1-2 stream nozzle that receives supply of steam from the steam generator and sprays a 1-2 stream of a gas phase containing a steam into the interior of the vaporization reactor.

17. The apparatus for vaporizing lactic acid according to claim 16, wherein:
the steam supply section further comprises a steam temperature adjusting part that adjusts the temperature of the 1-2 stream to 200 to 600°C.

18. The apparatus for vaporizing lactic acid according to claim 16, wherein:
the spray section is adjusted so that the 1-2 stream is sprayed at a flow rate of 0.1 g/min to 3.0 g/min.

19. The apparatus for vaporizing lactic acid according to claim 16, wherein:
the spray section is adjusted so that the 1-1 stream flow rate: the 1-2 stream flow rate = 1: 1.5 to 1: 5.

20. A method for vaporizing lactic acid comprising the steps of:
mixing and spraying a 3-1 stream of a liquid phase containing a lactic acid aqueous solution and a 3-2 stream of a gas phase containing a steam;
vaporizing the lactic acid aqueous solution through heat exchange between the 3-1 stream and the 3-2 stream; and
obtaining a 3-3 stream of a gas phase containing lactic acid single-molecule.

21. The method for vaporizing lactic acid according to claim 20, wherein:
the lactic acid aqueous solution contained in the 3-1 stream has a lactic acid concentration of 40 to 99 wt.%.

22. The method for vaporizing lactic acid according to claim 20, wherein:
the concentration of a multimer in the lactic acid aqueous solution contained in the 3-1 stream is 2 to 55 wt.%.

23. The method for vaporizing lactic acid according to claim 20, wherein:
a temperature of the 3-1 stream is 10 to 300°C.

24. The method for vaporizing lactic acid according to claim 20, wherein:
a temperature of the 3-2 stream is 200 to 600°C.

25. The method for vaporizing lactic acid according to claim 20, wherein:
a temperature difference between the 3-2 stream and the 3-1 stream is 200°C to 500°C.

26. The method for vaporizing lactic acid according to claim 20, wherein:
the 3-1 stream is sprayed at a flow rate of 0.05 g/min to 1.5 g/min.

27. The method for vaporizing lactic acid according to claim 20, wherein:
the 3-2 stream is sprayed at a flow rate of 0.1 g/min to 4.0 g/min.

28. The method for vaporizing lactic acid according to claim 20, wherein:
in the mixing and spraying step, the 3-1 stream flow rate: the 3-2 stream flow rate = 1: 1.5 to 1: 5.

29. A method for preparing acrylic acid, comprising the steps of:
obtaining lactic acid molecules by vaporizing lactic acid according to the method of any one of claims 20 to 28;
subjecting the lactic acid molecules to a dehydration reaction to prepare acrylic acid; and
obtaining the acrylic acid.

30. An apparatus for vaporizing lactic acid, comprising:
a feed supply section that supplies a 3-1 stream containing a lactic acid aqueous solution;
a steam supply section that supplies a 3-2 stream containing a steam;
a vaporization reaction section that receives supply of a 3-1 stream from the feed supply section and receives supply of a 3-2 stream from the steam supply section to perform a vaporization reaction of a lactic acid aqueous solution; and
an obtaining section that obtains a 3-3 stream containing vaporized lactic acid molecules.

31. The apparatus for vaporizing lactic acid according to claim 30, wherein:
the vaporization reaction section comprises a spray section that sprays a 3-1 stream supplied from the feed supply section and a 3-2 stream supplied from the steam supply section into the interior of the vaporization reaction section at the lower end.

32. The apparatus for vaporizing lactic acid according to claim 30, wherein:
the feed supply section comprises a lactic acid aqueous solution feed that supplies a lactic acid aqueous solution; and
a feed pretreatment section that adjusts the temperature and pressure of the 3-1 stream.

33. The apparatus for vaporizing lactic acid according to claim 32, wherein: the feed pretreatment section discharges the 3-1 stream at a temperature of 10 to 300°C.

34. The apparatus for vaporizing lactic acid according to claim 30, wherein:
the steam supply section comprises a water supply section that supplies water; and
a water pretreatment section that adjusts the temperature and pressure of the 3-2 stream.

35. The apparatus for vaporizing lactic acid according to claim 34, wherein:
the water pretreatment section discharges the 3-2 stream at a temperature of 200 to 600°C.

36. The apparatus for vaporizing lactic acid according to claim 31, wherein:
the spray section comprises a mix spray nozzle that mixes and sprays the 3-1 stream and the 3-2 stream.

37. The apparatus for vaporizing lactic acid according to claim 31, wherein:
the spray section comprises a 3-1 nozzle that sprays the 3-1 stream, and a 3-2 nozzle that sprays the 3-2 stream.

38. The apparatus for vaporizing lactic acid according to claim 31, wherein:
the spray section is adjusted so that the ratio of the 3-1 stream spray flow rate to the 3-2 stream spray flow rate is 1: 1.5 to 1: 5.

39. The apparatus for vaporizing lactic acid according to claim 30, wherein:
the obtaining section is located above the vaporization reaction section.

40. The apparatus for vaporizing lactic acid according to claim 30, wherein:
the obtaining section comprises a gas-liquid separator that separates and discharges vaporized lactic acid molecules and liquefied aqueous solution components.

41. The apparatus for vaporizing lactic acid according to claim 40, wherein:
the aqueous solution component discharged from the gas-liquid separator is recovered and reused as a lactic acid aqueous solution feed.
